Europäisches Patentamt

European Patent Office

Office européen des brevets

(19)

(11) Numéro de publication: **0 229 787**
**B1**

(12) **FASCICULE DE BREVET EUROPEEN**

(45) Date de publication du fascicule du brevet: **03.10.90**

(51) Int. Cl.⁵: **G 01 N 33/38**

(21) Numéro de dépôt: **86904059.2**

(22) Date de dépôt: **14.07.86**

(86) Numéro de dépôt international:
**PCT/CH86/00097**

(87) Numéro de publication internationale:
**WO 87/00636 29.01.87 Gazette 87/03**

(54) **PROCEDE ET DISPOSITIF AUTOMATIQUE DE MESURE DE LA TENEUR D'UN COMPOSANT SOLUBLE DANS UN PRODUIT PULVERULENT.**

(30) Priorité: **16.07.85 FR 8510987**

(43) Date de publication de la demande:
**29.07.87 Bulletin 87/31**

(45) Mention de la délivrance du brevet:
**03.10.90 Bulletin 90/40**

(84) Etats contractants désignés:
**CH DE FR GB IT LI**

(56) Documents cités:
**FR-A-2 485 733**
**FR-A-2 564 592**
**US-A-3 870 465**

(73) Titulaire: **ITECA S.A.**
**2, Plage de l'Estaque**
**F-13016 Marseille (FR)**

(72) Inventeur: **LIMON, Bernard**
**Rignat**
**F-01250 Ceyzeriat (FR)**

(74) Mandataire: **Nithardt, Roland**
**CABINET ROLAND NITHARDT Attn: Cabinet**
**MOSER & CIE Rue Edouard Verdan 15**
**CH-1400 Yverdon-les-Bains (CH)**

Courier Press, Leamington Spa, England.

EP 0 229 787 B1

## Description

La présente invention concerne un procédé de mesure de la teneur en chaux d'un produit pulvérulent, procédé dans lequel on introduit dans un récipient un volume déterminé du produit pulvérulent et un volume déterminé d'un solvant, on agite ce mélange de manière à dissoudre la chaux dans le solvant, et l'on mesure dans ce mélange une caractéristique électrique représentative de ladite teneur en chaux.

L'invention concerne également un dispositif de mesure de la teneur d'un composant soluble dans un produit pulvérulent, comportant au moins un récipient, des moyens pour doser une quantité donnée d'un solvant et l'amener à ce récipient, des moyens pour doser une quantité donnée du produit pulvérulent et l'amener audit récipient, des moyens pour agiter le contenu du récipient, des moyens pour mesurer dans le contenu du récipient une caractéristique électrique, et des moyens pour évacuer le contenu du récipient après cette mesure.

L'invention s'applique particulièrement, mais pas exclusivement, à la mesure de la teneur en chaux libre du ciment ou du clinker de ciment.

Dans les cimenteries, une telle mesure est faite régulièrement sur un échantillon de clinker prélevé à la sortie du four, puis refroidi et broyé, dans le but de régler la fabrication, en particulier le processus de cuisson dans le four.

Le brevet français No. 2 163 963 décrit un procédé et un dispositif pour effectuer cette mesure, d'une manière permettant la conduite automatique d'un four de cimenterie. On utilise dans ce cas un dispositif comprenant d'une part une enceinte, dans laquelle on prépare un mélange de l'échantillon de clinker broyé et de glycol chauffé et l'on mesure une caractéristique électrique de ce mélange, et d'autre part des moyens d'amenée de volumes dosés du clinker et du glycol vers cette enceinte. Les moyens de dosage et d'amenée du clinker broyé sont décrits plus en détail dans le brevet français No. 2 182 283 et comportent une coupelle calibrée, dans laquelle le clinker est déversé et tassé, puis arasé pour constituer le volume voulu. Cette coupelle est ensuite amenée mécaniquement par un bras mobile au-dessus de l'enceinte pour y déverser le clinker.

Ce mode opératoire et ce dispositif présentent cependant divers inconvénients qui affectent la précision des mesures et la fiabilité du processus, et donc aussi la qualité du clinker produit par le four dans le cas d'une conduite automatique. Tout d'abord, la masse volumique de l'échantillon de clinker broyé peut varier, en particulier avec la granulométrie de l'échantillon. Ceci ce produit notamment en cas de fonctionnement défectueux du four, produisant du clinker "incuit" dont la broyabilité diffère de celle du clinker normal. Il en résulte que la quantité de clinker introduite dans l'enceinte de mesure varie aussi et que la mesure est faussée. Or c'est précisément dans un tel cas que la fabrication doit être corrigée.

D'autre part, la détermination volumétrique des quantités avec ce dispositif ne permet pas de contrôle dans un processus automatique. Si l'un des appareils d'amenée fonctionne mal, la mesure peut s'effectuer sur des quantités fausses, par exemple si la coupelle calibrée n'est pas complètement remplie de clinker avant arasage. Il en va de même en cas de mauvais fonctionnement de la pompe à glycol.

En outre, l'enceinte de mesure est un instrument complexe de délicat. En particulier, sa vidange complète entre deux cycles de fonctionnement n'est pas toujours assurée, car les vannes peuvent être obstruées. Le déplacement de l'électrode mobile qu'elle renferme est aussi sujet à des défauts de fonctionnement.

Par conséquent, la présente invention a pour but de remédier à ces inconvénients, en fournissant un procédé du type mentionné en préambule et permettant d'obtenir une mesure précise et sûre de la teneur en chaux, au moyen d'un dispositif comportant des organes relativement simples et éprouvés. En particulier, l'invention doit assurer une fiabilité élevée des mesures successives effectuées au cours des cycles automatiques du fonctionnement du dispositif, afin d'être applicable dans un système de réglage automatique de la fabrication du produit pulvérulent, comme connu de FR—A—2 485 733 ou FR—A—2 564 592 public le 22 November 1985.

Dans ce but, le procédé de mesure selon l'invention est caractérisé en ce que l'on mesure le poids du produit pulvérulent introduit dans le récipient, par pesage du récipient avant et après l'introduction de ce produit, en ce que l'on calcule le rapport pondéral entre les volumes respectifs de produit pulvérulent et de solvant introduites dans le récipient, et en ce que l'on corrige en fonction de ce rapport le résultat de ladite mesure d'une caractéristique électrique. De préférence, on mesure le poids dudit volume de solvant également par pesage du récipient avant et après l'introduction du solvant.

Selon une forme de réalisation préférée du procédé, dans laquelle on effectue des cycles automatiques de mesure commandés par un calculateur électronique, ce calculateur reçoit des signaux représentatif des pesages, il calcule les poids respectifs du produit et du solvant, ainsi que ledit rapport pondéral, et il corrige ladite mesure d'une caractéristique électrique de manière à délivrer au moins un signal de sortie représentatif de la teneur en chaux du produit pulvérulent. Ce signal de sortie est utilisable notamment pour l'affichage du résultat de la mesure et/ou pour la conduite automatique de l'installation de fabrication du produit pulvérulent.

Avec cette forme préférée du procédé, on peut effectuer au moins un étalonnage du dispositif de pesage au cours du cycle automatique de mesure, le calculateur corrigeant les résultats des pesages en fonction de l'étalonnage. D'autre part, le calculateur peut modifier automatiquement un cycle de mesure si l'un des poids mesuré ou calculé se trouve en dehors d'une marge respective prédéterminée.

Le dispositif selon l'invention est agencé pour permettre la mise en oeuvre de ce procédé, il comporte des moyens pour déplacer le récipient et ist caractérisé par des moyens pour le peser.

Selon une forme préférée du dispositif, les moyens pour déplacer le récipient comprennent une pince agencée pour saisir le récipient et montée sur un support motorisé agencé pour déplacer le récipient le long d'un arc de cercle horizontal, en translation verticale et en pivotement autour d'un axe sensiblement horizontal.

De préférence, les moyens pour peser le récipient comportent une balance électronique. Selon une autre variante, ces moyens comportent un capteur à jauge de contrainte.

De préférence, les moyens pour doser une quantité donnée du solvant comportent une pompe volumétrique. De même, les moyens pour doser une quantité donnée du produit pulvérulent comportent un doseur volumétrique. Ce doseur volumétrique n'a pas besoin d'assurer un dosage extrêmement précis; il peut notamment être constitué avantageusement par un plateau rotatif équipé d'un racleur.

La présente invention et ses avantages seront mieux compris à l'aide de la description d'une forme de réalisation, prévue pour le dosage de la chaux libre dans un échantillon de clinker de ciment, après broyage de cet échantillon. Cette description est donnée ci-dessous à titre d'exemple non limitatif, en référence aux dessins annexés, dans lesquels:

La figure 1 est une vue schématique en plan du dispositif selon l'invention, et

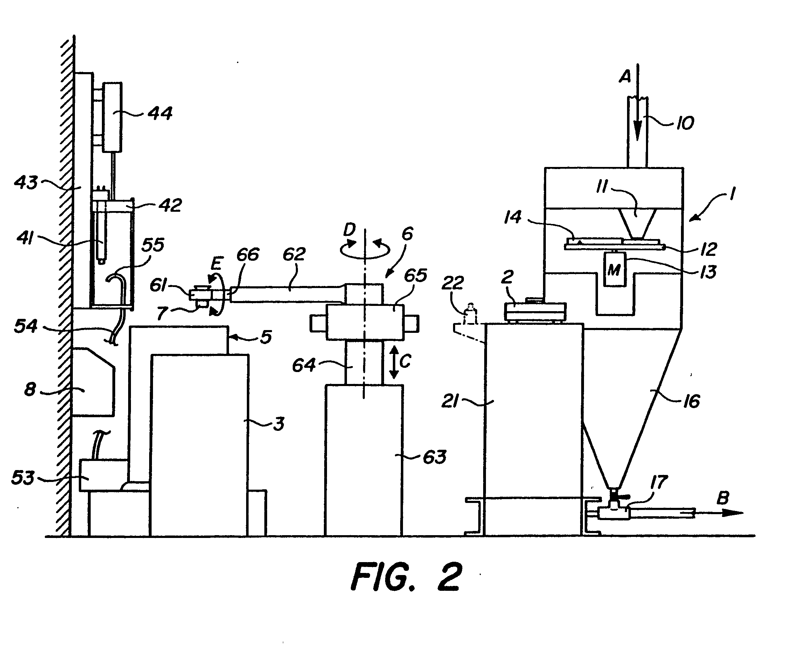

La figure 2 est une vue schématique en élévation du dispositif représenté sur la fig. 1.

En référence aux figures, le dispositif représenté comporte essentiellement un distributeur 1 de clinker broyé, une balance électronique 2, un bac d'évacuation 3, un bloc de mesure 4, un bloc d'alimentation en glycol 5 et un robot manipulateur 6 pour déplacer un récipient de mesure 7. Dans le cas présent, le récipient 7 est un bécher traditionnel, mais il est évident qu'il peut être constitué par n'importe quel type de récipient ouvert vers le haut et approprié à sa fonction. Le dispositif comprend en outre un pupitre de commande 8 équipé d'un calculateur 9.

Le distributeur 1 est raccordé à la sortie d'un conduit d'amenée 10 du clinker, par lequel les échantillons de clinker prélevés à la sortie du four, puis refroidis et broyés, arrivent suivant la flèche A, jusqu'à une trémie 11. En dessous de cette trémie se trouve un plateau rotatif 12 entraîné de manière intermittente par un moteur 13. Un racleur composé de deux éléments 14 et 15 est disposé dans une position fixe au-dessus de la face supérieure du plateau 12. Le premier élément 14 comporte un trou ou une encoche pour former sur le plateau un cordon de clinker de volume déterminé, tandis que le second élément 15 est disposé obliquement de manière à repousser ce cordon hors du plateau 12 et le déverser dans le récipient 7 amené en dessous. La matière excédentaire située devant l'élément 14 tombe dans une trémie d'évacuation 16, d'où elle est évacuée périodiquement dans la direction de la flèche B au moyen d'un éjecteur 17.

La balance 2 est une balance électronique de précision, d'une portée de l'ordre de 100 à 200 grammes avec un échelon de 1 mg. Elle est montée à une hauteur appropriée sur un support 21 et elle est raccordée électriquement au calculateur 9 pour lui transmettre les résultats de chaque pesage.

En variante, la balance 2 peut être remplacée par un capteur à jauge de contrainte, lequel doit alors être réétalonné automatiquement à chaque cycle de mesure, par dépôt d'un poids étalon 22 représenté en traits interrompus sur la figure.

Le bac d'évacuation 3 est ouvert vers le haut, de manière qu'on puisse y déverser facilement le contenu du récipient 7. Ce bac n'est représente que schématiquement; il peut être constitué par exemple par un simple tonneau qui est ouvert à son sommet et que l'on remplace périodiquement, ou par un réservoir plus complexe équipé de filtres et d'une pompe d'extraction pour le recyclage du glycol.

Le bloc d'alimentation en glycol 5 est disposé à proximité du bloc de mesure 4. Dans l'exemple illustré ici, il comporte un réservoir de glycol 51, monté sur un organe de chauffage 52 commandé par un thermostat, et une pompe volumétrique 53, par exemple une pompe péristaltique, qui est reliée au bloc de mesure 4 par une conduite 54 de distribution du glycol. Dans le bloc de mesure, l'extrémité de la conduite 54 est munie d'un bec 55 pour déverser le glycol chaud dans le récipient 7.

D'autre part, le bloc de mesure 4 comporte un ensemble d'instruments 41 destiné à être plongé dans le contenu du récipient 7 au cours du processus. A cet effet, l'ensemble 41 est monté sur un châssis 42 qui est mobile verticalement le long d'un support mural 43, grâce à un petit vérin 44 qui peut être par exemple un vérin électrique. L'ensemble 41 n'est représente que schématiquement, car il regroupe simplement plusieurs instruments dont chacun est bien connu. Il s'agit en l'occurence d'un agitateur destiné à mélanger dans le récipient 7 le glycol et le clinker pulvérulent, d'un régulateur de température comprenant une sonde thermique et un corps de chauffe, pour maintenir le mélange à une température précise et pour transmettre cette température aux organes de commande, et d'une paire d'électrodes agencées en vue de la mesure électrique prévue, qui est de préférence une mesure de la conductibilité du mélange, mais peut aussi être une mesure d'un potentiel, du pH ou d'une caractéristique similaire.

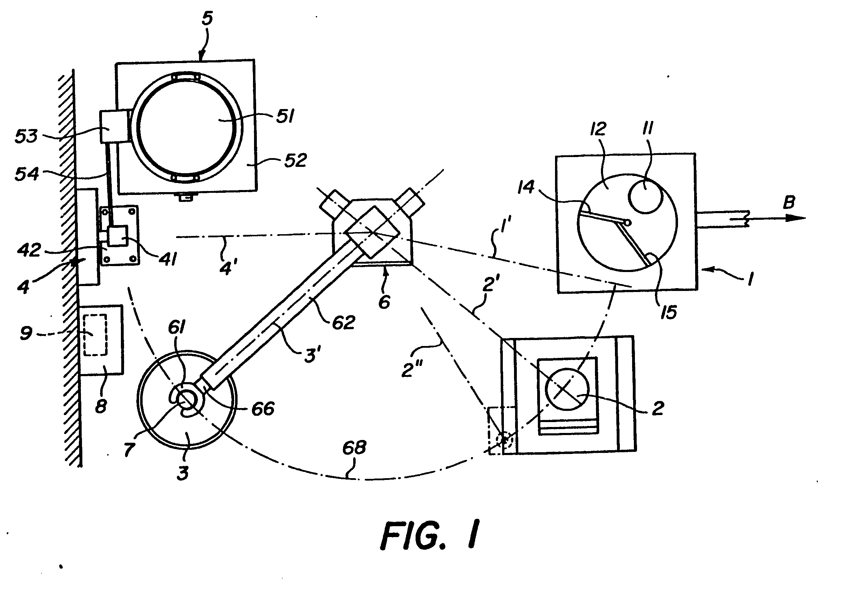

Comme on le décrira ci-dessous, le récipient 7 doit être déplacé plusieurs fois entre les appareils 1 à 4 au cours d'un cycle de mesure. A cet effet, il est porté par une pince 61 disposée à l'extrémité d'un bras horizontal 62 du robot manipulateur 6. Il d'agit d'un appareil à trois degrés de déplacement, d'un type bien connu dans les techniques de manutention et comportant essentiellement un socle fixe 63, une colonne verticale 64 mobile verticalement par rapport à ce socle, suivant la double flèche C, et le bras 62 monté au sommet de la colonne 64 de manière à pivoter autour de l'axe vertical de cette colonne, suivant la flèche D et sous l'action d'un mécanisme 65. L'extrémité libre 66 du bras 62 porte la pince 61 et elle est agencée pour pivoter autour de l'axe horizontal de ce bras, suivant la double flèche E. Un mécanisme d'ouverture et de fermeture de la pince 61 est logé dans le bras 62. Comme le montre la fig. 1, le robot manipulateur 6 est installé au centre d'un arc de cercle 68 le long duquel sont répartis les éléments 1 à 4 du dispositif. En plan, les déplacements du récipient 7 se font tous le long de cet arc de cercle. Sur la figure 1, les positions respectives du récipient 7 à proximité des différents appareils 1 à 4 sont indiquées par les positions correspondantes de l'axe du bras 62, indiquées par les références 1', 2', 3' et 4'. Une autre position 2'' est indiquée pour le cas de l'utilisation de l'étalon de poids 22.

Avec le procédé selon la présente invention, un cycle de fonctionnement du dispositif décrit ci-dessus se déroule comme suit, toutes les opérations de ce cycle étant commandées et contrôlées automatiquement à partir du pupitre de commande 8. Au repos, le récipient 7 est rempli de glycol et se trouve en position 3' au-dessus du bac d'évacuation 3. Par un pivotement de l'extrémité 66 du bras, il est renversé et vidé dans ce bac, puis amené en position 2'' pour être déposé sur la balance 2 et être taré. La balance transmet la tare du récipient au calculateur 9. Ensuite, le manipulateur 6 saisit à nouveau le récipient 7 par la pince 61 et l'amène en position 4' pour qu'il reçoive une dose de glycol chaud, par exemple 50 ml, délivrée par la pompe péristaltique 53 et la conduite 54. Le récipient est alors ramené en position 2'' pour être pesé sur la balance, le calculateur 9 calculant alors le poids exact de la dose de glycol par la différence des poids du récipient avant et après l'introduction du glycol. Ensuite, le récipient est amené en position 1' et le plateau rotatif 12 du distributeur 1 est mis en action pour une durée correspondant au déversement d'un volume donné de clinker pulvérulent dans le récipient, ce volume étant déterminé de manière à correspondre approximativement à un gramme de clinker. Pour déterminer le poids exact de la quantité de clinker introduite dans le récipient, ce dernier est pesé à nouveau sur la balance 2, le calculateur 9 déterminant à nouveau ce poids par différence entre les pesages avant et après. Le calculateur 9 calcule aussi le rapport entre le poids du clinker et le poids du glycol. Le récipient 7 est ramené en position 4' pour la mesure conductimétrique. L'ensemble d'instruments 41 est abaissé par le vérin 44 jusque dans le récipient, puis le régulateur de température est enclenché et l'agitateur est mis en fonction pour une durée prédéterminée. Ensuite, si la température du mélange se trouve dans la marge voulue, la mesure conductimétrique est effectuée et transmise au calculateur 9, qui la corrige en fonction du rapport pondéral entre le clinker et le glycol de délivre un signal de sortie, de préférence numérique, pour l'affichage des résultats sur le pupitre 8 et pour les organes de commande du four dans lequel est fabriqué le clinker. Ensuite, l'ensemble d'instruments 41 est remonté et le récipient 7 est ramené en position 3' pour déverser son contenu dans le bac 3 en vue du recyclage du glycol. Le récipient est ensuite rincé, en recevant en position 4' une nouvelle dose de glycol, qui peut être éventuellement remuée par l'agitateur et qui est ensuite déversée dans le bac 3, le récipient 7 restant alors en 3' en position de repos.

Dans le cas où la balance électronique 2 est remplacée par un capteur à jauge de contrainte, le cycle de fonctionnement comporte en outre au moins une phase d'étalonnage de ce capteur, au cours de laquelle le récipient 7 est déposé par la pince 61 dans une position d'attente, par exemple en position 1', tandis que cette pince est utilisée pour saisir l'étalon de poids 22 dans la position 2'', l'amener en position 2' pour être pesé, puis le remettre en place.

Le calculateur 9 détermine alors les poids du récipient, du glycol et du clinker en fonction du poids indiqué par le capteur pour l'étalon 22.

Avec le procédé décrit ci-dessus, comme tous les résultats des mesures sont transmis au calculateur 9, celui-ci peut effectuer des tests sur ces résultats et interrompre ou recommencer le cycle si l'une des valeurs sort d'une marge de tolérance prédéterminée. On évite ainsi les risques inhérents au dosage volumétrique selon l'art antérieur, avec lequel une insuffisance ou un excès de l'un ou l'autre des produits n'étaient pas détectés. Ce procédé permet de s'affranchir complètement de l'erreur de la prise d'échantillon dont on connaît le poids avec précision. Par ailleurs, la mesure peut être effectuée de façon répétitive sans que l'on prenne des précautions particulières pour que le poids de substances prélevées à chaque échantillonnage reste dans des limites prédéterminées. Un système dans lequel les échantillons dont le poids est trop éloigné d'une valeur déterminée, fixé par l'opérateur, sont écartés, est décrit dans le brevet français No. 2 564 592. L'inconvénient d'un tel système réside dans le fait que plusieurs échantillons peuvent éventuellement être rejetés successivement, par exemple si le récipient chargé de les recueillir ne se remplit qu'incomplètement suite à un défaut quelconque des organes de commande. Le rejet de plusieurs échantillons successifs entraîne la perte de mesures ce qui peut aboutir à des défauts de production, étant donné que les mesures sont exploitées pour assurer un contrôle automatique de la fabrication. Par ailleurs, dans le dispositif décrit ci-dessus, on utilise uniquement des appareils relativement simples et qui sont peu sujets à des perturbations. En outre, ces appareils se prêtent facilement à l'installation d'organes de contrôle de fonctionnement, par exemple des capteurs détectant la présence, l'absence ou la position du récipient 7, ainsi que les quantités de clinkers et du glycol encore disponibles. On obtient ainsi une très grande sécurité de fonctionnement et une excellente fiabilité des resultats des mesures.

Des essais ont montré que, dans une cimenterie, un dispositif selon l'invention, est à même d'effectuer un cycle toutes les dix minutes, donc de corriger la cuisson du clinker pratiquement en temps réel.

La présente invention n'est pas limitée à la forme de réalisation décrite ci-dessus à titre d'exemple, mais elle s'étend à toute modification ou variante évidente pour l'homme de l'art. En particulier, le dispositif n'est pas limité à la mesure de la teneur en chaux dans le clinker de ciment, mais il est utilisable, avec les modifications appropriées au cas particulier, pour n'importe quelle mesure de la teneur d'un composant soluble, dans un produit pulvérulent.

**Revendications**

1. Procédé de mesure de la teneur en chaux d'un produit pulvérulent, dans lequel on introduit dans un récipient un volume déterminé du produit pulvérulent et un volume d'un solvant, on agite ce mélange de manière à dissoudre la chaux dans le solvant, et l'on mesure dans ce mélange une caractéristique électrique représentative de ladite teneur en chaux, caractérisé en ce que l'on mesure le poids du produit pulvérulent introduit dans le récipient, par pesage du récipient avant et après l'introduction de ce produit, en ce que l'on calcule le rapport pondéral entre les volumes respectifs de produit pulvérulent et de solvant introduits dans le récipient, et en ce que l'on corrige en fonction de ce rapport le résultat de ladite mesure d'une caractéristique électrique.

2. Procédé selon la revendication 1, caractérisé en ce que l'on mesure le poids dudit volume de solvant également par pesage du récipient avant et après l'introduction du solvant.

3. Procédé selon la revendication 2, dans lequel on effectue des cycles automatiques de mesure commandés par un calculateur électronique, caractérisé en ce que ce calculateur reçoit des signaux représentatifs des pesages, calcule les poids respectifs du produit et du solvant, ainsi que ledit rapport pondéral, et corrige ladite mesure d'une caractéristique électrique de manière à délivrer au moins un signal de sortie représentatif de la teneur en chaux du produit pulvérulent.

4. Procédé selon la revendication 3, caractérisé en ce que l'on effectue au moins une étalonnage du dispositif de pesage au cours du cycle automatique de mesure, et en ce que le calculateur corrige les résultats des pesages en fonction de l'étalonnage.

5. Procédé selon la revendication 3, caractérisé en ce que le calculateur modifie automatiquement un cycle de mesure si l'un des poids mesurés ou calculés se trouve en dehors d'une marge de tolérance.

6. Dispositif de mesure de la teneur d'un composant soluble dans un produit pulvérulent, comportant au moins un récipient, des moyens pour déplacer ce récipient, des moyens pour doser un volume donné d'un solvant et l'amener à ce récipient, des moyens pour doser un volume donné du produit pulvérulent et l'amener au récipient, des moyens pour agiter le contenu du récipient, des moyens pour mesurer une caractéristique électrique dans le contenu du récipient, et des moyens pour évacuer le contenu du récipient après cette mesure, caractérisé en ce qu'il comporte des moyens (2) pour peser le récipient (7).

7. Dispositif selon la revendication 6, caractérisé en ce que les moyens pour déplacer le récipient comprennent une pince (61) agencée pour saisir le récipient (7) et monté sur un support motorisé (62) agencé pour déplacer le récipient le long d'un arc de cercle horizontal (68), en translation verticale (C) et en pivotement (E) autour d'un axe sensiblement horizontal.

8. Dispositif selon la revendication 6, caractérisé en ce que les moyens pour peser le récipient comportent une balance électronique (2).

9. Dispositif selon la revendication 6, caractérisé en ce que les moyens pour peser le récipient comportent un capteur à jauge de contrainte.

10. Dispositif selon l'une quelconque des revendications 6 à 9, caractérisé en ce que les moyens pour doser un volume donné d'un solvant comportent une pompe volumétrique (53).

11. Dispositif selon l'une quelconque des revendications 6 à 10, caractérisé en ce que les moyens pour doser un volume donné du produit pulvérulent comportent un doseur volumétrique (12 à 15).

12. Dispositif selon la revendication 11, caractérisé en ce que ledit doseur volumétrique comporte un plateau rotatif (12) équipé d'un racleur (14, 15).

**Patentansprüche**

1. Verfahren zum Messen des Kalkgehalts eines pulverartigen Produkts, bei welchem man in ein Gefäß ein bestimmtes Volumen des pulverartigen Produkts eingibt, sowie ein Volumen eines Lösungsmittels, dann diese Mischung so lange umrührt, bis der Kalk in dem Lösungsmittel aufgelöst ist und man dann in dieser Mischung einer für den Kalkgehalt typische elektrische Eigenschaft mißt, dadurch gekennzeichnet, daß man durch Wiegen des Gefäßes vor und nach der Eingabe dieses Produkts das Gewicht des in das Gefäß eingegeben pulverartigen Produkts mißt, daß man das gewwichtsanalytische Verhältnis zwischen den jeweiligen Volumina des pulverartigen Produkts und des Lösungsmittels, die man in das Gefäß eingebracht hat, berechnet, und daß man in Abhängigkeit dieses Verhältnisses das Ergebnis dieser Messung einer elektrischen Eigenschaft korrigiert.

2. Verfahren nach Anspruch 1, dadurch gekennzeichnet, daß man das Gewicht des Lösungsmittelvolumens ebenfalls durch Wägung vor und nach der Eingabe des Lösungsmittels mißt.

3. Verfahren nach Anspruch 2, bei welchem man von einem elektronischen Rechner gesteuerte, automatisch ablaufende Meßzyklen durchführt, dadurch gekennzeichnet, daß dieser Rechner den Wägungen entsprechende typische Signale erhält, dann die jeweiligen Gewichte für das Produkt und das Lösungsmittel sowie das gewichtsanalytische Verhältnis errechnet und diese Messung einer elektrischen Eigenschaft korrigiert, um mindestens ein typisches Ausgangssignal für den Kalkgehalt des pulverartigen Produkts zu liefern.

4. Verfahren nach Anspruch 3, dadurch gekennzeichnet, daß man während des automatischen Meßzyklus mindestens eine Eichung der Wiegevorrichtung durchführt, und daß der Rechner die Ergebnisse der Wägungen in Abhängigkeit der Eichung korrigiert.

5. Verfahren nach Anspruch 3, dadurch gekennzeichnet, daß der Rechner automatische einen Meßzyklus abändert, wenn sich eines der gemessenen oder berechneten Gewichte außerhalb eines Toleranzbereichs befindet.

6. Vorrichtung zum Messen des Gehalts an einem löslichen Bestandteil in einem pulverartigen Produkt, die folgendes aufweist: mindestens ein Gefäß, Einrichtungen, um dieses Gefäß örtlich zu versetzen, Einrichtungen, um ein bestimmtes Volumen eines Lösungsmittels zu dosieren und in dieses Gefäß einzugeben, Einrichtungen, um ein bestimmtes Volumen des pulverartigen Produkts zu dosieren und es in das Gefäß einzugeben, Einrichtungen, um den Inhalt des Gefäßes, umzurühren, Einrichtungen, um eine elektrische Eigenschaft in dem Inhalt des Gefäßes zu messen und Einrichtungen, um den Inhalt des Gefäßes nach dieser Messung auszuleeren, dadurch gekennzeichnet, daß sie Einrichtungen (2) zum Wiegen des Gefäßes (7) aufweist.

7. Vorrichtung nach Anspruch 6, dadurch gekennzeichnet, daß die Einrichtungen zum Umsetzen des Gefäßes eine Klemmvorrichtung (61) aufweisen, die dazu dient, das Gefäß (7) zu ergreifen und die an einem motorgetriebenen Halter (62) befestigt ist, der dazu dient, das Gefäß längs eines horizontalen Kreisbogens (68) zu bewegen, des weiteren in einer Vertikalbewegung (C), und in einer Drehbewegung (E) um eine im wesentlichen horizontale Achse.

8. Vorrichtung nach Anspruch 6, dadurch gekennzeichnet, daß die Einrichtungen zum Wiegen des Gefäßes eine elektronische Waage (2) aufweisen.

9. Vorrichtung nach Anspruch 6, dadurch gekennzeichnet, daß die Einrichtungen zum Wiegen des Gefäßes ein Meßgerät mit Drehnungsmeßstreifen aufweisen.

10. Vorrichtung nach einem der Ansprüche 6 bis 9, dadurch gekennzeichnet, daß die Einrichtungen zum Dosieren eines bestimmten Volumens eines Lösungsmittels eine Verdrängerpumpe (53) aufweisen.

11. Vorrichtung nach einem der Ansprüche 6 bis 10, dadurch gekennzeichnet, daß die Einrichtungen zum Dosieren eines bestimmten Volumens des pulverartigen Produkts ein volumetrisches Dosiergerät (12—15) aufweisen.

12. Vorrichtung nach Anspruch 11, dadurch gekennzeichnet, daß dieses volumetrisches Dosiergerät einen mit einem Abstreifer (14, 15) ausgestatteten Teller (12) aufweist.

**Claims**

1. A process for measuring the chalk content of a powdery product, into which a determined volume of the powdery product and a volume of a solvent is introduced into a container, this mixture is stirred so as to dissolve the chalk in the solvent, and in this mixture an electrical characteristic representative of said chalk content is measured, characterised in that the weight of the powdery product introduced into the container is measured, by weighing the container before and after the introduction of this product, in that the weighable ratio between the respective volumes of the powdery product and solvent introduced into the container is calculated, and in that the result of said measurement of an electrical characteristic is corrected as a function of this ratio.

2. A process according to Claim 1, characterised in that the weight of said volume of solvent is also measured by weighing the container before and after the introduction of the solvent.

3. A process according to Claim 2, in which automatic measurement cycles controlled by an electronic calculator are carried out, characterised in that this calculator receives signals representative of the weighing operations, calculates the respective weights of the product and of the solvent, as well as said weighable ratio, and corrects said measurement of an electrical characteristic so as to emit at least one output signal representative of the chalk content of the powdery product.

4. A process according to Claim 3, characterised in that at least one calibration of the weighing device is carried out during the automatic measurement cycle, and in that the calculator corrects the results of the weighing operations as a function of the calibration.

5. A process according to Claim 3, characterised in that the calculator automatically modifies a measurement cycle if one of the weights measured or calculated lies outside a tolerance margin.

6. A device for measuring the content of a soluble component in a powdery product, comprising at least one container, means for moving said container, means for determining a given volume of a solvent and conveying it to the container, means for determining a given volume of the powdery product and conveying it to the container, means for stirring the contents of the container, means for measuring an electrical characteristic in the contents of the container, and means for discharging the contents of the container after this measurement, characterised in that it comprises means (2) for weighing the container (7).

7. A device according to Claim 6, characterised in that the means for moving the container comprise a clamp (61) designed to grasp the container (7) and mounted on a motorised support (62) designed to move the container along a horizontal arc (68) of a circle, in a vertical movement (C) and a pivoting movement (E) around a substantially horizontal axis.

8. A device according to Claim 6, characterised in that the means for weighing the container comprise electronic scales (2).

9. A device according to Claim 6, characterised in that the means for weighing the container comprise a strain gauge pick-up.

10. A device according to any one of Claims 6 to 9, characterised in that the means for determining a given volume of a solvent comprise a volumetric pump (53).

11. A device according to any one of Claims 6 to 10, characterised in that the means for determining a given volume of the powdery product comprise a volumetric dosimeter (12 to 15).

12. A device according to Claim 11, characterised in that said volumetric dosimeter comprises a rotary plate (12) equipped with a scraper (14, 15).

FIG. I

1

FIG. 2

EP 0 229 787 B1